# EUROPEAN PATENT SPECIFICATION

(11) **EP 3 158 973 B1**
(45) Date of publication and mention of the grant of the patent: **26.12.2018**
(21) Application number: 16275153.1
(22) Date of filing: 12.10.2016
(51) Int. Cl.: A61F 2/01

(54) **METHOD OF MANUFACTURE OF A BIODEGRADABLE VASCULAR FILTER**
VERFAHREN ZUR HERSTELLUNG EINES BIOABBAUBARERN GEFÄSSFILTER
PROCÉDÉ DE FABRICATION D'UN FILTRE VASCULAIRE BIODÉGRADABLE

(30) Priority: 19.10.2015 GB 201518450
(43) Date of publication of application: 26.04.2017
(73) Proprietor: Cook Medical Technologies LLC, Bloomington, IN 47404 (US)
(72) Inventor: Klausen, Kasper, 4623 Lille Skensved (DK)
(74) Representative: Williams Powell

(56) References cited:
- WO-A1-2010/082189
- WO-A2-2008/010197
- WO-A2-2014/140088
- US-A1- 2008 188 887
- US-A1- 2010 042 135
- US-A1- 2013 226 222

## Description

The present invention relates to a method of manufacture of a vascular filter.

Filtering devices that are percutaneously placed in the vena cava have been available for a number of years. A need for filtering devices arises in trauma patients, orthopaedic surgery patients, neurosurgery patients, or in patients having medical conditions requiring bed rest or non-movement because of the likelihood of thrombosis in the peripheral vasculature of patients, or for use in thrombectomy therapy. A thrombus may break away from the vessel wall, and, depending on the size of the thrombus, may pose a serious risk of pulmonary embolism when blood clots migrate from the peripheral vasculature through the heart and into the lungs. Vascular filters are also known for other medical indications and filtering needs.

A problem with known filters that are only required to be implanted temporarily is that removal from the patient requires a further medical procedure, and all of the risks involved. To avoid this, some implantable medical devices are at least partially biodegradable. Examples of such medical devices are disclosed in US 2003/0153972, US 2007/0161968, US 2007/0232169, US 2008/0208321, US 2009/0026650, US 2009/0267259, US 2010/0172953, US 2012/0089221, US 6,214,040, US 6,338,739, and US 8,298,466. Bioabsorbable polymers made by Zeus, Inc under the trade mark Absorv® (www.zeusinc.com/advanced-products/absorv-bioabsorbables) are examples of suitable materials for making such biodegradable medical devices.

However, this technology has not generally been extended to vascular filters, due to problems with controlling the degradation of the structure of the device.

WO 2008010197 discloses a method of manufacturing a vascular filter device from a cone shaped film.

WO 2014/140088 discloses a vascular filter device.

The present invention seeks to provide an improved vascular filter.

According to an aspect of the present invention, there is provided a method of manufacturing a vascular filter, including: providing a generally tubular member including a first portion and a second portion, the second portion being arranged generally concentrically around the first portion, wherein the material for the first portion has a faster biodegradability rate than the material for the second portion; cutting the first portion to form filtration struts for a filtration basket; and cutting the second portion to form supporting stent struts.

In an embodiment, the first portion and the second portion are co-extruded to form the generally tubular member. This enables a filter having portions with different degradation profiles to be made without the need for joining or connection processing, or the need for any glue or additive.

The first portion and the second portion may include or consist of materials having different mechanical properties, for example, different stiffness or elasticity.

The first portion and the second portion may be at least partially spaced from one another in a radial direction.

The first portion may include a plurality of (for example, at least four) connecting members that bridge a space between the first portion and the second portion.

The first portion and the second portion may be separately cut to form the filtration basket and the stent struts.

The materials may be polymers.

The method may be a method of manufacturing a vascular filter as defined below.

According to another aspect of the present invention, there is provided a vascular filter including a support portion provided with a plurality of supporting stent struts and a filtration portion provided with a plurality of filtration struts, the support portion being arranged concentrically around at least a proximal end of the filtration portion and the filtration portion being attached to the support portion and forming a filtration basket, wherein the filtration portion includes a material having a faster biodegradability rate than the support portion.

The vascular filter has a simple filter structure that can biodegrade in a reliable manner. The vascular filter has portions having different degradation profiles.

The vascular filter may be a vena cava filter. In particular it may be a vascular filter for implantation in the inferior vena cava.

In an embodiment, both the filtration portion and the support portion are biodegradable. The entire filter is preferably biodegradable.

The filtration portion may include a hub to which the plurality of filtration struts is connected.

The hub may have the same biodegradability rate as the filtration struts or a faster biodegradability than the filtration struts.

Each filtration strut may be attached at its proximal end to the support portion. In an embodiment, therefore, the filtration struts are attached at their proximal ends to the support portion and at their distal ends to the hub.

In an embodiment, the filtration portion extends longitudinally beyond an end of the support portion.

The filtration portion and the support portion may have different mechanical properties, for example, different stiffness or elasticity. In an embodiment the support portion has a higher stiffness to provide higher radial force.

The vascular filter may be cut from a tube comprising an inner concentric portion and an outer concentric portion, the inner concentric portion including a material having a faster biodegradability rate than a material of the outer concentric portion.

In an embodiment, the inner concentric portion and the outer concentric portion may be at least partially spaced from one another in a radial direction.

A plurality of connecting members may bridge a space between the concentric portions and connect the concentric portions together. In certain embodiments, at least four connecting members are provided.

The connecting members may have a faster biodegradability rate than the outer concentric portion.

The connecting members may have the same biodegradability rate as the inner concentric portion or a slower biodegradability rate than the inner concentric portion.

The inner concentric portion may be cut to form a hub at which the plurality of filtration struts is connected and/or the inner concentric portion may be cut to form the plurality of filtration struts.

The filtration struts may be formed by the connecting members.

The vascular filter may be manufactured by a method as specified above.

Embodiments of the present invention are described below, by way of example only, with reference to the accompanying drawings, in which:
Figure 1 is a transverse sectional view of a tube from which an embodiment of a vascular filter may be formed;
Figure 2 is a representation of a vascular filter that has been formed from the tube of Figure 1;
Figure 3 is a view of the distal end of the tube of Figure 1 showing (as dotted lines) cuts that may be made to form the vascular filter of Figure 2;
Figure 4 is a view of the proximal end of the tube of Figure 1 showing (as dotted lines) cuts that may be made to form the vascular filter of Figure 2;
Figure 5 is a perspective view of the tube of Figure 1 showing (as dotted lines) cuts that may be made to form the vascular filter of Figure 2;
Figure 6 is a partial view of the tube of Figure 5 in a partially expanded state;
Figures 7 to 9 illustrate the cutting and expansion of a single flange of the tube of Figures 1 and 5;
Figure 10 is a perspective view of a tube from which an embodiment of a vascular filter may be formed;
Figure 11 is an end view of the tube of Figure 10;
Figure 12 is a partial view of the tube of Figure 10 in an expanded state;
Figure 13 is a perspective view of a tube from which an embodiment of a vascular filter may be formed;
Figure 14 is an end view of the tube of Figure 13;
Figure 15 is a view of the tube of Figures 13 and 14 after cutting; and
Figure 16 is a view of the vascular filter that has been formed from the tube of Figures 13 and 14.

It is to be understood that the Figures are schematic and do not show the various components in their actual scale. In many instances, the Figures show scaled up components to assist the reader.

In this description, the term distal, when used with respect to the filter or a component thereof, denotes an end that it downstream with respect to blood flow. The term proximal is used to denote an end that is upstream with respect to blood flow.

As used herein, the term "biodegradable" is intended to encompass the terms "bioresorbable" and "bioerodable". Any portion of a medical device of the present invention that is described herein as "biodegradable", "bioresorbable", or "bioerodable" will, over time, lose bulk mass by being degraded, resorbed or eroded by normal biological processes in the body. The prefix "bio" indicates that the erosion occurs under physiological conditions, as opposed to other erosion processes, caused, for example, by high temperature, strong acids or bases, UV light or weather conditions. A biodegradable material has the ability naturally to disappear over time *in vivo* in accordance with any biological or physiological mechanism, such as, for example, erosion, degradation, dissolution, chemical depolymerisation including at least acid- and base-catalysed hydrolysis and free radical-induced depolymerisation, enzymatic depolymerisation, absorption and/or resorption within the body. Typically, the material is metabolised or broken down by normal biological processes into metabolites or break-down products that are substantially non-toxic to the body and are capable of being resorbed and/or eliminated through normal excretory and metabolic processes of the body. As such, biodegradable devices do not require surgical removal.

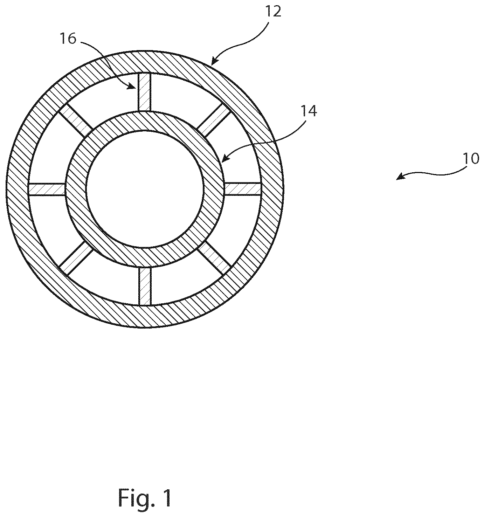

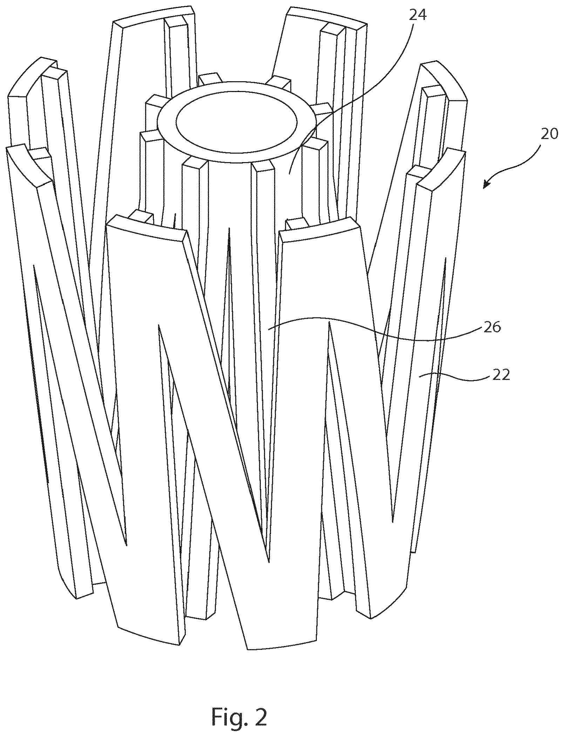

Figure 1 illustrates a tube 10 (hereinafter "intermediate tube 10"), which is an intermediate structure in the formation of a vascular filter 20 such as that illustrated in Figure 2. The intermediate tube 10 includes an outer tube 12 surrounding and radially spaced from an inner tube 14 so as to have a gap therebetween. The outer tube 12 and the inner tube 14 are connected by, in this embodiment, eight connecting flanges 16. The intermediate tube 10 in this embodiment has a diameter of approximately 3 to 6mm (for example 4 to 6mm), a wall thickness of approximately 1 to 2mm, and a length in the range of approximately 40 to 65mm

In this embodiment, the outer tube 12 is formed from L-polylactide (L-PLA), which is a biodegradable polymer. The inner tube 14 and the connecting flanges 16 are formed from a co-polymer of PLA, which may, for example, be a co-polymer of PLA, polyglycolic acid and/or polycaprolactone, that has a faster degradation profile than L-PLA. The intermediate tube 10 is preferably formed from the two polymers by co-axial extrusion of the tubular structure using a suitably shaped die. Co-extrusion of the materials having the different degradation profiles avoids problems with joining processing of the different parts of the device. By co-extruding the materials into a tube and cutting the filtration and support elements therefrom, there is no requirement for an adhesive or other additive for joining or connection processing.

Figure 2 illustrates a vascular filter that has been laser cut from an intermediate tube 10 as illustrated in Figure 1. Stent struts 22 are cut from the outer tube 12 to form a supporting stent structure of the vascular filter 20. An uncut portion at the distal end of the inner tube 14 forms a hub 24. The connecting flanges 16 are cut to form filtration struts 26 that extend from their distal ends at the hub 24 to their proximal ends where they connect to the proximal end of the supporting stent structure formed by the stent struts 22. It can be seen, therefore, that the vascular filter 20 includes a filtration basket formed by the filtration struts 26 arranged inside the supporting stent structure formed by the stent struts 22.

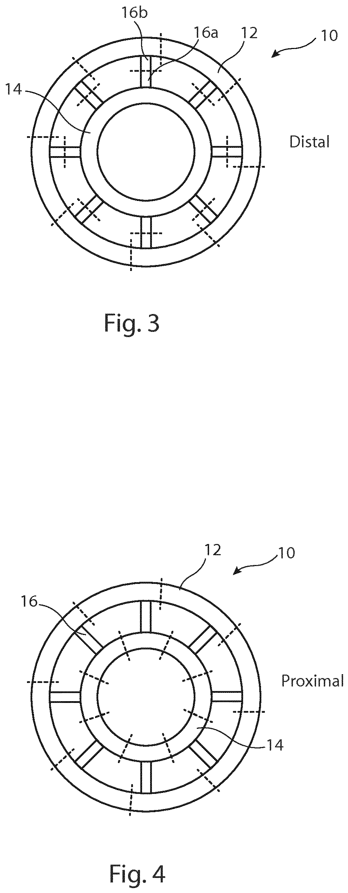

Figures 3 to 9 explain in more detail how the intermediate tube 10 may be cut to form the vascular filter 20 shown in Figure 2.

Figures 3 to 5 illustrate how the intermediate tube 10 of Figure 1 can be cut, for example using a laser, to enable the vascular filter 20 of Figure 2 to be formed. Each flange 16 undergoes a transverse cut starting from its distal end as shown by the dotted lines. The cut extends longitudinally along the length of each flange 16 so that a portion 16a of the flange remains attached to the inner tube 14, and a portion 16b remains attached to the outer tube 12. The cut does not extend all the way to the proximal end of the flange 16. A short portion at the proximal end is left uncut so that the two flange portions (16a, 16b) remain attached to one another.

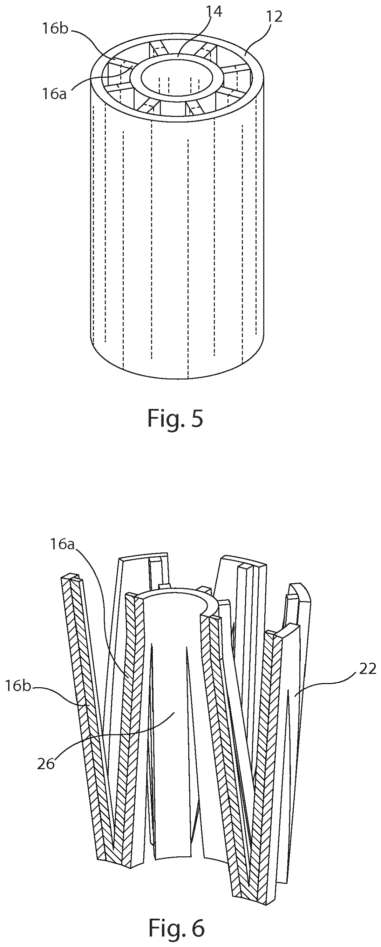

In the next step, the outer tube 12 is cut longitudinally either side of each flange 16. As illustrated in Figure 5, the cuts do not extend the entire length of the outer tube 12, but terminate before the end. However, the cuts alternate with regard to whether they leave an uncut portion at the proximal end or at the distal end of the outer tube 12.

As a final step, the inner tube 14 is cut longitudinally, starting at the proximal end and terminating before the distal end. The uncut portion of the inner tube 14 thus remains circumferentially intact, therefore forming a hub 24 at the distal end as can best be seen in Figure 6.

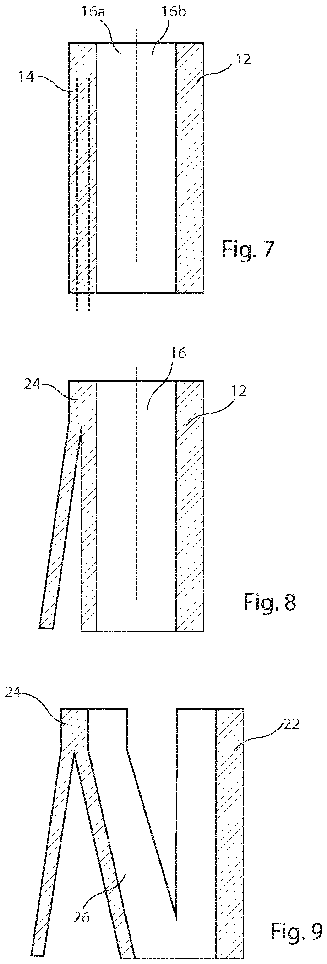

Figures 7 to 9 also illustrate the cutting of the flanges 16. An individual flange 16 is shown extending between the inner tube 14 and the outer tube 12 (which is shown in cross-section). As described above, a cut extends through the flange 16 to create two flange portions 16a, 16b.

At this stage, the vascular filter 20 is expandable, as shown in Figure 6. The portions of the outer tube 12 between the cuts can expand to form stent struts 22. The flanges 16 extend diagonally from the hub 24 to the expanded stent struts 22 formed from the outer tube 12 to form filtration struts 26. Sections of the inner tube 14 extend diagonally away from the hub 24 with the flange portions 16a to which they are attached. The filter legs 26 are thus formed partially from the flange portions 16a, and partially from sections of the inner tube 14.

The above-described cutting method is merely exemplary. The skilled person would appreciate that other cutting patterns could be used to form the vascular filter 20 from the intermediate tube 10.

As indicated above, the inner tube 14 and the connecting flanges 16 of the intermediate tube 10 are formed from a polymer having a faster degradation profile than the outer tube 12. This results in the vascular filter 20 illustrated in Figure 2 including a hub 24 and filtration struts 26 being more biodegradable (biodegrading more quickly) than the stent struts 22 of the supporting stent structure. Additionally, in this embodiment, the surface of the hub 24 is abraded using any suitable technique, such as sand-blasting or etching. This ensures that the hub 24 starts to degrade even before the filtration struts 26. Other methods of achieving this, such as other surface treatment methods and/or sizing the hub 24 appropriately are also possible.

The vascular filter 20 is thermal processed in an expanded configuration according to techniques available to the skilled person. It is then compressed ready for use.

In use, the vascular filter 20 is delivered to the inferior vena cava using techniques well known in the art. The vascular filter 20 may be guided to its *in vivo* location using a wire running through its inner lumen. Upon expansion of the device *in vivo*, the supporting stent structure formed by the stent struts 22 expands to engage against the vessel wall. This assists in holding the filtration basket in place and in the alignment thereof. The filtration basket may only be required by the patient for a limited period of time, for example a few weeks or a few months (for example, 1 to 3 months). After this period of time, the hub 24 and then the filtration struts 26 biodegrade and break down into lactic acid that can be readily metabolised and excreted by the patient. The supporting stent structure formed by the stent struts 22 remains in place during this process, ensuring that the hub 24 and filtration struts 26 degrade first and do not break away from their location in the vessel before having degraded sufficiently, and provides support to the vessel wall for some time thereafter (for example several months) before biodegrading itself.

Of course, there are many modifications that could be made to the above-described embodiment. The arrangement of connecting flanges 16 shown in Figure 1 is merely exemplary. Other arrangements may have fewer connecting flanges, such as four or five. Of course many other arrangements of connecting flanges 16 may be envisaged.

In the embodiments described above, the entire vascular filter 20 is biodegradable. In a modification, the vascular filter 20 may be only partially biodegradable. For example, the outer tube of the intermediate tube 10 may be made from a non-biodegradable material. This would result in a vascular filter 20 in which after biodegradation of the hub 24 and the filtration struts 26, a permanent supporting stent structure formed from the stent struts 22 remains within the patient's vasculature. In some examples, the connecting flanges 16 may have a slower degradation profile than the hub 24, which may or may not be the same as that of the outer tube 12. The degradation may be controlled by a combination of selecting suitable materials and processing thereof.

The filtration struts 26 could be formed with portions of both slower degradation rate material and faster degradation rate material.

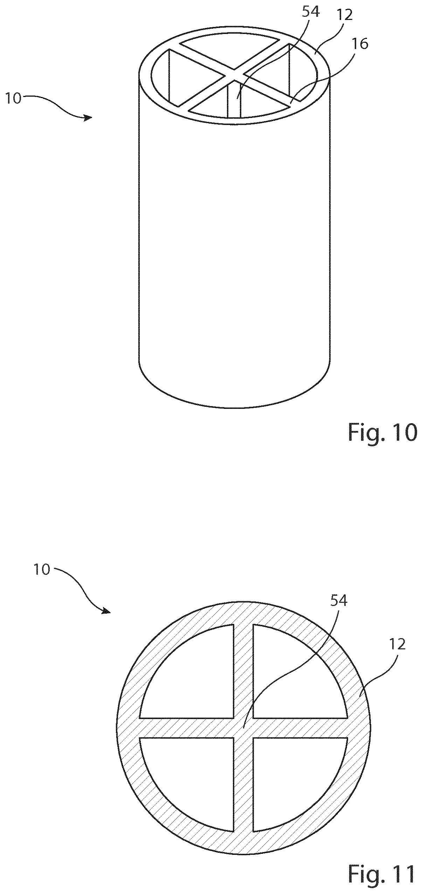

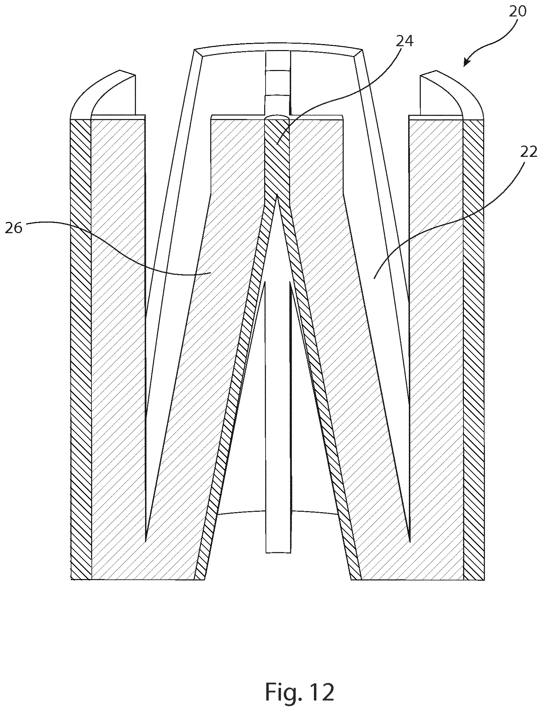

Figures 10 and 11 illustrate an intermediate tube 10 from which a modified vascular filter 20 may be formed. In this modification, there is no inner tube, but rather, a central rod 54 surrounded by an outer tube 12. In the illustrated embodiment, four connecting flanges 16 are shown. In this modification, a filter can be formed from the intermediate tube in the same way as described above with respect to Figures 1 and 2. The intermediate tube 10 illustrated in Figures 10 and 11 can be cut in a manner analogous to that described above for the intermediate tube 10 of Figure 1, and expanded to form a vascular filter as illustrated in Figure 12.

The skilled person will appreciate that the filter could be formed from other polymeric and non-polymeric materials. The outer tube 12 of the intermediate tube 10 could be non-biodegradable and made of a self-expanding material or of a balloon expandable material. Suitable materials are well known in the art and include, in the case of balloon expandable materials: steel, titanium, nickel and others. Self-expanding materials include spring steel or a shape memory alloy or polymer. In one preferred embodiment, the outer tube 12 of the intermediate tube 10 is made of a shape memory alloy based on nickel and titanium (for instance Nitinol). This results in a vascular filter 20 in which the stent struts 22 do not biodegrade.

Preferably, however, the stent struts 22 are designed to degrade gradually over time, for instance over a period of months or years. For this purpose, the outer tube 12 of the intermediate tube 10 may be made of an alloy of nickel and titanium with magnesium or iron. This results in a structure that will slowly biodegrade, typically over a period of many months, or one or more years, thereby resulting in complete removal of the stent struts 22 from the patient over time.

The inner tube 14, rod 54 and/or connecting flanges 16 of the intermediate tube 10 is preferably of a biodegradable polymer, such as: poly-L,D-lactide, poly-L-lactide, poly-D-lactide, bioglass, poly(alpha hydroxy acid), polyglycolic acid, polylactic acid, polycaprolactone, polydioxanone, polyglucanate, polylactic acid-polyethylene oxide copolymers, tyrosine-derived polycarbonate, polyglycolide, modified cellulose, collagen, poly(hydroxybutyrate), polyanhydride, polyphosphoester, poly(amino acids) or combinations thereof.

The materials having different degradation profiles could be different materials. In some embodiments the materials could be molecularly the same but have different molecular densities (for example, a higher molecular density reducing the degradation rate), different molecular weights, different crystallinities or different water absorption properties, could be different blends of the base material, or could have additives that change their rate of degradation. Other ways of causing materials to have different degradation profiles will be known to the person skilled in the art.

The preferred features and modifications described with respect to the embodiment of vascular filter 20 illustrated in Figure 2 are equally applicable as appropriate to the vascular filter 20 illustrated in Figure 12.

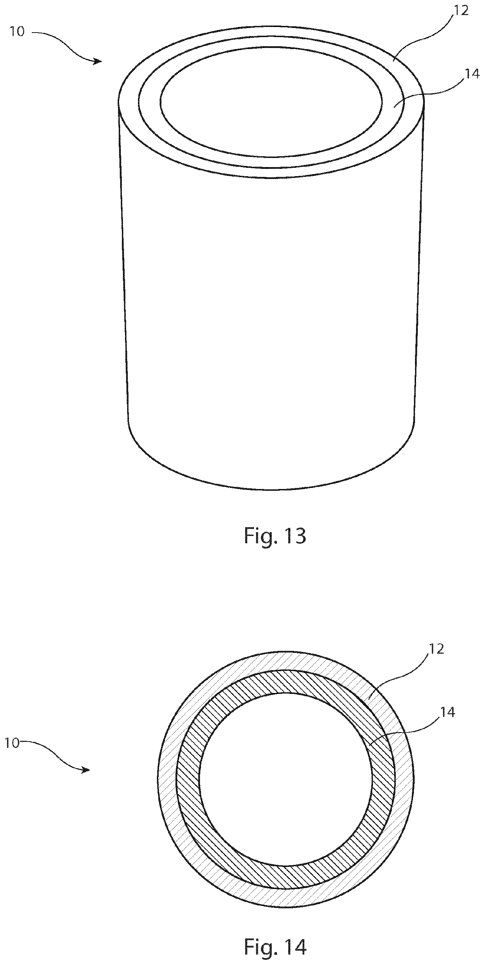

Another embodiment of vascular filter is illustrated in Figures 13 to 16. Figures 13 and 14 illustrate an intermediate tube 10 analogous to that illustrated in Figures 1 and 10. The intermediate tube 10 includes an outer tube 12 formed from a material that biodegrades less quickly than the material from which the inner tube 14 is made. In the embodiment illustrated in Figures 13 and 14, the outer tube 12 and the inner tube 14 are adjacent one another and are not radially spaced from one another. As described with the previous embodiments, the outer tube 12 and the inner tube 14 are preferably co-extruded to form the intermediate tube 10.

In order to form a vascular filter 20 from the intermediate tube 10 illustrated in Figures 13 and 14, the portion of the outer tube 12 at the distal end of the intermediate tube 10 is stripped away, for example, by laser ablation, to expose the inner tube 14.

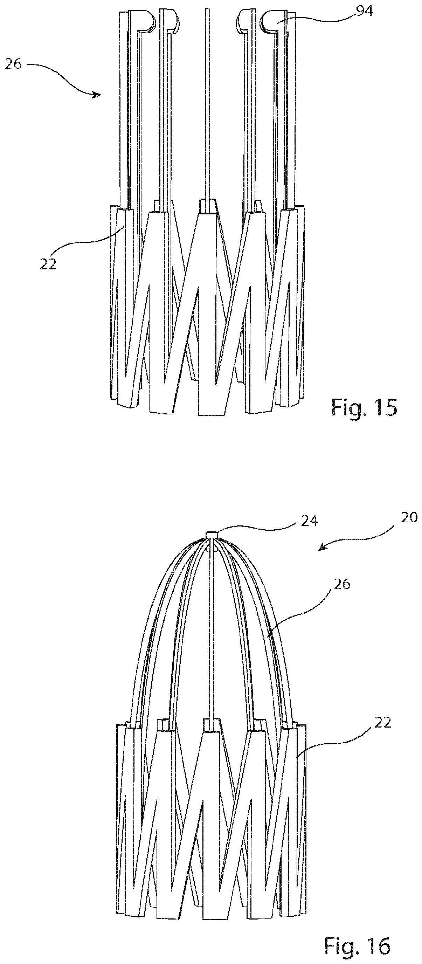

At the distal end, cuts are made through the exposed inner tube 14 to form unconnected filtration struts 26 having, in this embodiment, connecting lugs 94 at their distal ends. At the proximal end of the intermediate tube 10, cuts are made through both the outer tube 12 and the inner tube 14, and then the tube may be expanded to form the structure illustrated in Figure 15. Finally, the filtration struts 26 are connected together at their distal ends by way of the connecting lugs 94, which are, for example, welded or soldered together, to form a hub 24. The device may then be compressed for delivery as described with earlier embodiments.

All details regarding materials and modifications of the embodiments illustrated in Figures 1 to 12 are equally applicable to the embodiment of Figures 13 to 16.

As indicated above, the embodiment illustrated in Figures 13 to 16 has no spacing between the outer tube 12 and the inner tube 14. In a modification, there may be a small space between the outer tube 12 and the inner tube 14, and connecting flanges 16 or similar structure may be provided to join these together.

It can be seen that what has been described is a vascular filter that may only temporarily be required by a patient. By selection of suitable materials for forming the hub, the filtration basket and the supporting stent structure, the vascular filter biodegrades (partially or fully), with the hub, or the hub and filtration struts, biodegrading before the supporting stent structure.

The filter can have two or more degradation stages and biodegrades in a reliable manner.

What has been described and illustrated herein is a preferred embodiment of the invention along with some of its variations. The terms, descriptions and Figures used herein are set forth by way of illustration only and are not meant as limitations. Those skilled in the art will recognise that many variations are possible within the scope of the invention, which is intended to be defined by the following claims in which all terms are meant in their broadest reasonable sense unless otherwise indicated.

## Claims

1. A method of manufacturing a vascular filter (20) from a generally tubular member (10), the generally tubular member including a first portion (14) and a second portion (12), the second portion being arranged generally concentrically around the first portion, wherein the material for the first portion has a faster biodegradability rate than the material for the second portion; the method including:
cutting the first portion to form filtration struts (26) for a filtration basket; and
cutting the second portion to form supporting stent struts (22).

2. A method as claimed in claim 1, wherein the first portion (14) and the second portion (12) are co-extruded to form the generally tubular member (10).

3. A method as claimed in claim 1 or 2, wherein the first portion (14) and the second portion (12) are at least partially spaced from one another in a radial direction.

4. A method as claimed in claim 3, wherein the first portion (14) includes a plurality of connecting members (16) that bridge a space between the first portion and the second portion (12), optionally wherein the filtration struts (26) are formed by cutting the connecting members.

5. A method as claimed in any preceding claim, wherein the first portion (14) and the second portion (12) are separately cut to form the filtration basket and the stent struts (22).

6. A method as claimed in any preceding claim, wherein the materials are polymers.

7. A method as claimed in any preceding claim, wherein the vascular filter (20) includes a support portion formed by the supporting stent struts (22), the support portion being arranged concentrically around at least a proximal end of the filtration basket, and the filtration basket being attached to the support portion.

8. A method as claimed in claim 7, wherein the filtration basket and the support portion of the vascular filter (20) are biodegradable, and/or wherein the vascular filter is biodegradable.

9. A method as claimed in claim 7 or 8, wherein the filtration basket of the vascular filter (20) includes a hub (24) to which the plurality of filtration struts (26) is connected.

10. A method as claimed in claim 9, wherein the hub (24) of the vascular filter (20) has the same biodegradability rate as the filtration struts (26) or wherein the hub of the vascular filter has a faster biodegradability rate than the filtration struts.

11. A method as claimed in any of claims 7 to 10, wherein each filtration strut (26) is attached at its proximal end to the support portion of the vascular filter (20).

12. A method as claimed in any of claims 7 to 11, wherein the filtration basket extends longitudinally beyond an end of the support portion of the vascular filter.

13. A method as claimed in any of claims 7 to 12, wherein the filtration struts (26) have a faster biodegradability rate than the support portion of the vascular filter (20).

14. A method as claimed in any of claims 7 to 13, wherein the first portion (14) is cut to form a hub (24) at which the plurality of filtration struts (26) is connected.

15. A method as claimed in claim 14, wherein the filtration struts (26) have the same biodegradability rate as the hub (24) or wherein the filtration struts have a slower biodegradability rate than the hub.

## Patentansprüche

1. Verfahren zur Herstellung eines Gefäßfilters (20) aus einem allgemein röhrenförmigen Element (10), wobei das allgemein röhrenförmige Element einen ersten Abschnitt (14) und einen zweiten Abschnitt (12) aufweist, wobei der zweite Abschnitt allgemein konzentrisch um den ersten Abschnitt angeordnet ist, wobei das Material für den ersten Abschnitt eine schnellere biologisch Abbaurate als das Material für den zweiten Abschnitt aufweist; wobei das Verfahren das Folgende aufweist:
Schneiden des ersten Abschnitts zur Bildung von Filterstreben (26) für einen Filterkorb;
und
Schneiden des zweiten Abschnitts zur Bildung von unterstützenden Stentstreben (22).

2. Verfahren nach Anspruch 1, wobei der erste Abschnitt (14) und der zweite Abschnitt (12) zur Bildung des allgemein röhrenförmigen Elements (10) koextrudiert werden.

3. Verfahren nach Anspruch 1 oder 2, wobei der erste Abschnitt (14) und der zweite Abschnitt (12) in einer radialen Richtung zumindest teilweise voneinander beabstandet sind.

4. Verfahren nach Anspruch 3, wobei der erste Abschnitt (14) eine Vielzahl von Verbindungselementen (16) aufweist, die einen Raum zwischen dem ersten Abschnitt und dem zweiten Abschnitt (12) überbrücken, wobei die Filterstreben (26) fakultativ durch Schneiden der Verbindungselemente ausgebildet werden.

5. Verfahren nach einem der vorhergehenden Ansprüche, wobei der erste Abschnitt (14) und der zweite Abschnitt (12) zur Bildung des Filterkorbs und der Stentstreben (22) getrennt geschnitten werden.

6. Verfahren nach einem der vorhergehenden Ansprüche, wobei die Materialien Polymere sind.

7. Verfahren nach einem der vorhergehenden Ansprüche, wobei der Gefäßfilter (20) einen Stützabschnitt aufweist, der von den unterstützenden Stentstreben (22) gebildet wird, wobei der Stützabschnitt konzentrisch zumindest um ein proximales Ende des Filterkorbs angeordnet ist und der Filterkorb am Stützabschnitt befestigt ist.

8. Verfahren nach Anspruch 7, wobei der Filterkorb und der Stützabschnitt des Gefäßfilters (20) biologisch abbaubar sind und/oder wobei der Gefäßfilter biologisch abbaubar ist.

9. Verfahren nach Anspruch 7 oder 8, wobei der Filterkorb des Gefäßfilters (20) einen Ansatz (24) aufweist, mit dem die Vielzahl von Filterstreben (26) verbunden ist.

10. Verfahren nach Anspruch 9, wobei der Ansatz (24) des Gefäßfilters (20) dieselbe biologische Abbaurate wie die Filterstreben (26) aufweist oder wobei der Ansatz des Gefäßfilters eine schnellere biologische Abbaurate als die Filterstreben aufweist.

11. Verfahren nach einem der Ansprüche 7 bis 10, wobei jede Filterstrebe (26) an ihrem proximalen Ende am Stützabschnitt des Gefäßfilters (20) befestigt ist.

12. Verfahren nach einem der Ansprüche 7 bis 11, wobei sich der Filterkorb längs jenseits eines Endes des Stützabschnitts des Gefäßfilters erstreckt.

13. Verfahren nach einem der Ansprüche 7 bis 12, wobei die Filterstreben (26) eine schnellere biologische Abbaurate als der Stützabschnitt des Gefäßfilters (20) aufweisen.

14. Verfahren nach einem der Ansprüche 7 bis 13, wobei der erste Abschnitt (14) zur Bildung eines Ansatzes (24), mit dem die Vielzahl von Filterstreben (26) verbunden ist, geschnitten wird.

15. Verfahren nach Anspruch 14, wobei die Filterstreben (26) dieselbe biologische Abbaurate wie der Ansatz (24) aufweisen oder wobei die Filterstreben eine langsamere biologische Abbaurate als der Ansatz aufweisen.

## Revendications

1. Procédé de fabrication d'un filtre vasculaire (20) à partir d'un élément essentiellement tubulaire (10), l'élément essentiellement tubulaire comprenant une première partie (14) et une seconde partie (12), la seconde partie étant agencée d'une façon essentiellement concentrique autour de la première partie, dans lequel le matériau constituant la première partie présente une vitesse de biodégradabilité supérieure à celle du matériau constituant la seconde partie; le procédé comprenant les étapes suivantes:
couper la première partie de manière à former des entretoises de filtration (26) pour un panier de filtration; et
couper la seconde partie de manière à former des entretoises de stent de support (22).

2. Procédé selon la revendication 1, dans lequel la première partie (14) et la seconde partie (12) sont co-extrudées pour former l'élément essentiellement tubulaire (10).

3. Procédé selon la revendication 1 ou 2, dans lequel la première partie (14) et la seconde partie (12) sont au moins partiellement espacées l'une de l'autre dans une direction radiale.

4. Procédé selon la revendication 3, dans lequel la première partie (14) comprend une pluralité d'éléments de connexion (16) qui relient un espace entre la première partie et la seconde partie (12), optionnellement dans lequel les entretoises de filtration (26) sont formées en coupant les éléments de connexion.

5. Procédé selon l'une quelconque des revendications précédentes, dans lequel la première partie (14) et la seconde partie (12) sont coupées séparément de manière à former le panier de filtration et les entretoises de stent (22).

6. Procédé selon l'une quelconque des revendications précédentes, dans lequel les matériaux sont des polymères.

7. Procédé selon l'une quelconque des revendications précédentes, dans lequel le filtre vasculaire (20) comprend une partie de support formée par les entretoises de stent de support (22), la partie de support étant agencée de façon concentrique autour d'au moins une extrémité proximale du panier de filtration, et le panier de filtration étant attaché à la partie de support.

8. Procédé selon la revendication 7, dans lequel le panier de filtration et le partie de support du filtre vasculaire (20) sont biodégradables, et/ou dans lequel le filtre vasculaire est biodégradable.

9. Procédé selon la revendication 7 ou 8, dans lequel le panier de filtration du filtre vasculaire (20) comprend un moyeu (24) auquel la pluralité d'entretoises de filtration (26) sont connectées.

10. Procédé selon la revendication 9, dans lequel le moyeu (24) du filtre vasculaire (20) présente la même vitesse de biodégradabilité que les entretoises de filtration (26), ou dans lequel le moyeu du filtre vasculaire présente une vitesse de biodégradabilité supérieure à celle des entretoises de filtration.

11. Procédé selon l'une quelconque des revendications 7 à 10, dans lequel chaque entretoise de filtration (26) est attachée à son extrémité proximale à la partie de support du filtre vasculaire (20).

12. Procédé selon l'une quelconque des revendications 7 à 11, dans lequel le panier de filtration s'étend de façon longitudinale au-delà d'une extrémité de la partie de support du filtre vasculaire.

13. Procédé selon l'une quelconque des revendications 7 à 12, dans lequel les entretoises de filtration (26) présentent une vitesse de biodégradabilité supérieure à celle de la partie de support du filtre vasculaire (20) .

14. Procédé selon l'une quelconque des revendications 7 à 13, dans lequel la première partie (14) est coupée de manière à former un moyeu (24) auquel la pluralité d'entretoises de filtration (26) sont connectées.

15. Procédé selon la revendication 14, dans lequel les entretoises de filtration (26) présentent la même vitesse de biodégradabilité que le moyeu (24), ou dans lequel les entretoises de filtration présentent une vitesse de biodégradabilité inférieure à celle du moyeu.
